# EUROPEAN PATENT APPLICATION

(11) **EP 4 116 320 A1**
(43) Date of publication of application: **11.01.2023**
(21) Application number: 21829276.1
(22) Date of filing: 16.06.2021
(51) Int. Cl.: C07K 14/78, C12N 5/07, C12N 1/00, A61L 27/02, A61L 27/24, A61L 27/36, A61L 27/38, A61L 27/44, A61L 27/50, A61L 27/52, A61L 27/58

(54) **GEL COMPOSITION AND PRODUCTION METHOD THEREFOR, AND THREE-DIMENSIONAL TISSUE BODY AND PRODUCTION METHOD THEREFOR**

(30) Priority: 22.06.2020 JP 2020107245; 14.10.2020 JP 2020173465
(71) Applicant: TOPPAN INC., Tokyo 110-0016 (JP); OSAKA UNIVERSITY, Suita-shi Osaka 565-0871 (JP)
(72) Inventor: KITANO Shiro, Tokyo 110-0016 (JP); MATSUSAKI Michiya, Suita-shi, Osaka 565-0871 (JP); SAKURAI Hidehiro, Suita-shi, Osaka 565-0871 (JP); UETAKE Yuta, Suita-shi, Osaka 565-0871 (JP)
(74) Representative: TBK
(86) International application number: PCT/JP2021/022923
(87) International publication number: WO 2021/261355

(57) **Abstract**

The present invention relates to a gel composition containing at least one selected from the group consisting of an extracellular matrix component and a fragmented extracellular matrix component, and an ion of a metal element.

## Description

### Technical Field

The present invention relates to a gel composition and a production method for the same. The present invention further relates to a three-dimensional tissue body in which the gel composition according to the present invention is used, and a production method for the same.

### Background Art

Gels of extracellular matrix components such as collagen are commonly used as scaffold materials when culturing cells. A collagen gel is usually prepared by a technique of dissolving collagen under an acidic condition, and thereafter performing neutralization and heating at about 37°C to gelate collagen. In addition, a technique of gelling collagen by crosslinking it using a crosslinking agent such as formaldehyde or glutaraldehyde is also known.

Meanwhile, for example, Patent Literature 1 discloses an extracellular matrix-containing composition containing a fragmented extracellular matrix component in which an extracellular matrix component is fragmented, and an aqueous medium. The extracellular matrix-containing composition disclosed in Patent Literature 1 shows a thermo-reversible sol-gel transition, which makes gelation and solation by temperature control possible.

### Citation List

### Patent Literature

[Patent Literature 1] PCT International Publication No. WO2019/208831

### Summary of Invention

### Technical Problem

Conventional techniques for producing a collagen gel have problems such as a cytotoxicity issue due to an acidic condition or a crosslinking agent, difficulty in controlling an elastic modulus, and difficulty in obtaining a gel having a high elastic modulus. On the other hand, in a method of utilizing the fragmented extracellular matrix component disclosed in Patent Literature 1, there is no cytotoxicity issue, controlling an elastic modulus is also possible to a certain extent, and a gel having a high elastic modulus can be obtained. However, with the method disclosed in Patent Literature 1, the obtained gel was opaque, making it difficult to observe the inside.

An object of the present invention is to provide a gel composition which is transparent, has a high elastic modulus, and contains an extracellular matrix component and/or a fragmented extracellular matrix component.

### Solution to Problem

The present invention relates to a gel composition containing at least one selected from the group consisting of an extracellular matrix component and a fragmented extracellular matrix component, and an ion of a metal element.

A gel composition according to the present invention is transparent and has a high elastic modulus because the gel composition contains an extracellular matrix component and/or a fragmented extracellular matrix component and also contains an ion of a metal element. The gel composition according to the present invention is based on the finding that a gel which is transparent and has a high elastic modulus (meaning hard) can be obtained by bringing a solution containing the extracellular matrix component and/or the fragmented extracellular matrix component into contact with (adding it dropwise to) a solution containing the ion of a metal element.

In the above-mentioned gel composition, the above-mentioned metal element is preferably at least one metal element selected from the group consisting of transition metal elements and base metal elements, is more preferably at least one metal element selected from the group consisting of transition metals of Group 10, transition metals of Group 11, and metals of Group 12 of the periodic table, and is further preferably at least one selected from the group consisting of copper, zinc, palladium, platinum, and gold. Thus, the above-mentioned effect is exhibited more significantly.

In the above-mentioned gel composition, the above-mentioned extracellular matrix component preferably contains collagen. Thus, the above-mentioned effect is exhibited more significantly.

In the above-mentioned gel composition, the fragmented extracellular matrix component preferably contains fragmented collagen. Thus, the above-mentioned effect is exhibited more significantly.

In the above-mentioned gel composition, at least one selected from the group consisting of the above-mentioned extracellular matrix component and the above-mentioned fragmented extracellular matrix component may be crosslinked.

The present invention further relates to a production method for a gel composition, the method including a step of bringing a solution containing at least one selected from the group consisting of an extracellular matrix component and a fragmented extracellular matrix component into contact with a solution containing an ion of a metal element.

According to the production method for a gel composition according to the present invention, a gel composition which is transparent and has a high elastic modulus (meaning hard) can be obtained.

In the above-mentioned production method for a gel composition, the above-mentioned metal element is preferably at least one metal element selected from the group consisting of transition metal elements and base metal elements, is more preferably at least one metal element selected from the group consisting of transition metals of Group 10, transition metals of Group 11, and metals of Group 12 of the periodic table, and is further preferably at least one selected from the group consisting of copper, zinc, palladium, platinum, and gold. Thus, the above-mentioned effect is exhibited more significantly.

In the above-mentioned production method for a gel composition, the above-mentioned extracellular matrix component preferably contains collagen. Thus, the above-mentioned effect is exhibited more significantly.

In the above-mentioned production method for a gel composition, the fragmented extracellular matrix component preferably contains fragmented collagen. Thus, the above-mentioned effect is exhibited more significantly.

In the production method for a gel composition, the above-mentioned contacting step may be performed at -3°C or higher and 10°C or lower. Thus, an excessive reaction can be avoided, which makes it possible to efficiently obtain the gel composition.

The present invention still further relates to a production method for a three-dimensional tissue body, the method including: a step of obtaining a cell-containing gel composition by bringing at least one selected from the group consisting of an extracellular matrix component and a fragmented extracellular matrix component, cells, and an ion of a metal element into contact with each other in an aqueous medium; and a step of culturing the above-mentioned cell-containing gel composition.

According to the above-mentioned production method for a three-dimensional tissue body, a three-dimensional tissue body having a transparent appearance can be obtained. Therefore, the cells and the like inside the three-dimensional tissue body can be easily observed. In addition, according to the above-mentioned production method for a three-dimensional tissue body, the three-dimensional tissue body having a high elastic modulus can be obtained.

In the above-mentioned production method for a three-dimensional tissue body, the above-mentioned step of obtaining the cell-containing gel composition may include bringing a suspension containing the at least one selected from the group consisting of the above-mentioned extracellular matrix component and the above-mentioned fragmented extracellular matrix component, the above-mentioned cells, and a first aqueous medium into contact with a solution containing the above-mentioned ion of the metal element and a second aqueous medium.

In the above-mentioned production method for a three-dimensional tissue body, the above-mentioned step of culturing may be performed under a condition in which at least some of the above-mentioned cells maintain a viable state.

In the above-mentioned production method for a three-dimensional tissue body, the method may further include a step of incubating the above-mentioned cell-containing gel composition at 20°C to 30°C after the above-mentioned step of obtaining the cell-containing gel composition and before the above-mentioned step of culturing.

In the above-mentioned production method for a three-dimensional tissue body, the metal element may be at least one metal element selected from the group consisting of transition metal elements and base metal elements.

The present invention still further relates to a three-dimensional tissue body composition containing: at least one selected from the group consisting of an extracellular matrix component and a fragmented extracellular matrix component; cells; and an ion of a metal element, in which the three-dimensional tissue body is transparent at 37°C.

In the above-mentioned three-dimensional tissue body, a total light transmittance at 37°C may be 80% or more.

In the above-mentioned three-dimensional tissue body, the metal element may be at least one metal element selected from the group consisting of transition metal elements and base metal elements.

### Advantageous Effects of Invention

According to the present invention, it is possible to provide a gel composition which is transparent, has a high elastic modulus, and contains an extracellular matrix component and/or a fragmented extracellular matrix component.

### Brief Description of Drawings

FIG. 1 is a photograph showing the results of Test Example 1.
FIG. 2 is a photograph showing the results of Test Example 2.
FIG. 3 is a photograph showing the results of Test Example 3.
FIG. 4 shows photographs showing the results of Test Example 4.
FIG. 5(A) is a photograph showing the appearance of a three-dimensional tissue body produced in Test Example 6, and FIG. 5(B) is a photograph showing the inside thereof.
FIG. 6 is a graph showing the elastic modulus of a gel composition produced from a solution of each metal ion.
FIG. 7(A) is a graph showing the transmittance at the wavelength of 500 nm of gel compositions produced from a fragmented collagen solution and a Pt²⁺ solution at each concentration. FIG. 7(B) is an image observed from the top surface of gels produced from the fragmented collagen solution and the Pt²⁺ solution at each concentration.
FIG. 8 shows photographs showing the results of Test Example 9.

### Description of Embodiments

Embodiments for implementing the present invention will be described in detail below. However, the present invention is not limited to the following embodiments.

### [Gel composition]

A gel composition according to the present embodiment contains at least one selected from the group consisting of an extracellular matrix component and a fragmented extracellular matrix component, and an ion of a metal element.

The extracellular matrix component is an assembly of extracellular matrix molecules which is formed by a plurality of extracellular matrix molecules. The extracellular matrix molecule may be a substance that is present outside the cells in a multicellular organism. Any substance can be used as the extracellular matrix molecule as long as it does not adversely affect cell growth and formation of cell agglomerates. Examples of the extracellular matrix molecules include, but are not limited to, collagen, laminin, fibronectin, vitronectin, elastin, tenascin, entactin, fibrillin, and proteoglycan. For the extracellular matrix component, one type of these extracellular matrix molecules may be used alone, or two or more types thereof may be used in combination.

The extracellular matrix component may contain collagen or may be composed of collagen, for example. When the extracellular matrix component contains collagen, the effect of the present invention that the gel composition is transparent and the elastic modulus of the gel composition is high (meaning hard) can be exhibited more significantly. In addition, when the extracellular matrix component contains collagen, the collagen functions as a scaffold for cell adhesion when the gel composition according to the present embodiment is used as a scaffold material when culturing cells, thereby further promoting the formation of a three-dimensional cell structure.

The extracellular matrix molecules may be modified bodies and variants of the above-mentioned extracellular matrix molecules, or may be polypeptides such as chemically synthesized peptides. The extracellular matrix molecule may have repeats of a sequence represented by Gly-X-Y characteristic of collagen. Gly represents a glycine residue, and X and Y each independently represent any amino acid residue. A plurality of Gly-X-Y's may be the same as or different from each other. Having the repeats of the sequence represented by Gly-X-Y reduces constraints on the arrangement of molecular chains, thereby obtaining the gel composition according to the present embodiment in which the function as a scaffold material when culturing cells is better, for example. In the extracellular matrix molecule having the repeats of the sequence represented by Gly-X-Y, the proportion of the sequence represented by Gly-X-Y in the total amino acid sequence may be 80% or more, and is preferably 95% or more. Alternatively, the extracellular matrix molecule may be a polypeptide having an RGD sequence. The RGD sequence refers to a sequence represented by Arg-Gly-Asp (arginine residue-glycine residue-aspartic acid residue). Having the RGD sequence further promotes cell adhesion, making the gel composition according to the present embodiment more suitable as a scaffold material when culturing cells, for example. Examples of extracellular matrix molecules containing the sequence represented by Gly-X-Y and the RGD sequence include collagen, fibronectin, vitronectin, laminin, and cadherin.

Examples of collagen include fibrillar collagen and non-fibrillar collagen. The fibrillar collagen means collagen that is the main component of collagen fibers, and specific examples thereof include type I collagen, type II collagen, and type III collagen. Examples of the non-fibrillar collagen include type IV collagen.

Examples of proteoglycans include, but are not limited to, chondroitin sulfate proteoglycans, heparan sulfate proteoglycans, keratan sulfate proteoglycans, and dermatan sulfate proteoglycans.

The extracellular matrix component may contain at least one selected from the group consisting of collagen, laminin, and fibronectin, and preferably contains collagen because this makes the effect of the present invention more remarkable. The collagen is preferably fibrillar collagen and more preferably type I collagen. Commercially available collagen may be used as the fibrillar collagen, and specific examples thereof include porcine skin-derived type I collagen manufactured by NH Foods Ltd.

The extracellular matrix component may be an animal-derived extracellular matrix component. Examples of animal species from which the extracellular matrix component is derived include, but are not limited to, humans, pigs, and bovines. For the extracellular matrix component, a component derived from one kind of animal may be used, or components derived from a plurality of kinds of animals may be used in combination.

The fragmented extracellular matrix component can be obtained by fragmenting the above-mentioned extracellular matrix component. The term "fragmenting" means reducing agglomerates of extracellular matrix molecules to a smaller size. Fragmenting may be performed under the condition of cleaving bonds within the extracellular matrix molecules, or under the conditions of not cleaving bonds within the extracellular matrix molecules. The fragmented extracellular matrix component may contain an extracellular matrix component which has been defibrated (defibrated extracellular matrix component), which is a component in which the above-described extracellular matrix component has been defibrated by applying physical force. Defibration is one aspect of fragmentation, and is performed under the condition of not cleaving bonds within extracellular matrix molecules, for example.

A method for fragmenting the extracellular matrix component is not particularly limited. As a method for defibrating the extracellular matrix component, for example, the extracellular matrix component may be defibrated by applying physical force such as an ultrasound homogenizer, a stirring homogenizer, or a high-pressure homogenizer. When using the stirring homogenizer, the extracellular matrix component may be homogenized as it is, or may be homogenized in an aqueous medium such as physiological saline. In addition, a millimeter-size or nanometer-size defibrated extracellular matrix component can be obtained by adjusting the homogenization time, number of times, and the like. The defibrated extracellular matrix component can also be obtained by defibration by repeating freezing and thawing.

The fragmented extracellular matrix component may at least partially contain the defibrated extracellular matrix component. In addition, the fragmented extracellular matrix component may be composed of only the defibrated extracellular matrix component. That is, the fragmented extracellular matrix component may be the defibrated extracellular matrix component. The defibrated extracellular matrix component preferably contains a collagen component which has been defibrated (defibrated collagen component). The defibrated collagen component preferably maintains the triple helical structure derived from collagen. The defibrated collagen component may be a component that partially maintains the triple helical structure derived from collagen.

Examples of the shape of the fragmented extracellular matrix component include a fibrous shape. The fibrous shape means a form constituted of filamentous collagen components, or a form constituted of filamentous extracellular matrix components crosslinked between molecules. At least a part of the fragmented extracellular matrix component may be fibrous. The fibrous extracellular matrix component includes a thin filamentous product (fibrils) formed by agglomeration of a plurality of filamentous extracellular matrix molecules, a filamentous product formed by further agglomeration of fibrils, a product in which these filamentous products have been defibrated, and the like. The RGD sequence is preserved without disruption in the fibrous extracellular matrix component.

The average length of the fragmented extracellular matrix component may be 100 nm or more and 400 µm or less, or may be 100 nm or more and 200 µm or less. In one embodiment, the average length of the fragmented extracellular matrix component may be 5 µm or more and 400 µm or less, may be 10 µm or more and 400 µm or less, may be 22 µm or more and 400 µm or less, or may be 100 µm or more and 400 µm or less. In another embodiment, the average length of the fragmented extracellular matrix component may be 100 µm or less, may be 50 µm or less, may be 30 µm or less, may be 15 µm or less, may be 10 µm or less, may be 1 µm or less, or may be 100 nm or more from the viewpoint of better redispersibility. The average length of the most part of the fragmented extracellular matrix component among the entire fragmented extracellular matrix component is preferably in the above-mentioned numerical value range. Specifically, the average length of 95% of the fragmented extracellular matrix component among the entire fragmented extracellular matrix component is preferably in the above-mentioned numerical value range. The fragmented extracellular matrix component is preferably a fragmented collagen component having the average length in the above-mentioned range, and is more preferably a defibrated collagen component having the average length in the above-mentioned range.

The average diameter of the fragmented extracellular matrix component may be 10 nm or more and 30 µm or less, may be 30 nm or more and 30 µm or less, may be 50 nm or more and 30 µm or less, may be 100 nm or more and 30 µm or less, may be 1 µm or more and 30 µm or less, may be 2 µm or more and 30 µm or less, may be 3 µm or more and 30 µm or less, may be 4 µm or more and 30 µm or less, or may be 5 µm or more and 30 µm or less. The fragmented extracellular matrix component is preferably a fragmented collagen component having the average diameter in the above-mentioned range, and is more preferably a defibrated collagen component having the average diameter in the above-mentioned range.

The average length and the average diameter of the fragmented extracellular matrix component can be obtained by measuring each individual part of the fragmented extracellular matrix component with an optical microscope to perform image analysis. In the present specification, the term "average length" means the average value of the length of a measured sample in a longitudinal direction, and the term "average diameter" means the average value of the length of a measured sample in a direction orthogonal to the longitudinal direction.

The fragmented extracellular matrix component usually becomes an opaque gel when being gelated, but a transparent gel is formed even when the gel composition according to the present invention contains the fragmented extracellular matrix component.

In the gel composition according to the present embodiment, at least a part of the extracellular matrix component and/or the fragmented extracellular matrix component (hereinafter collectively referred to as "extracellular matrix component and the like") may be intermolecularly or intramolecularly crosslinked. The extracellular matrix component and the like may be crosslinked within the molecules constituting the extracellular matrix component and the like, or may be crosslinked between the molecules constituting the extracellular matrix component and the like.

The form of crosslinking of the extracellular matrix component and the like may be at least partly attributable to the formation of hydrogen bonds between carboxyl groups and the like of extracellular matrix molecules and ions of a metal element. Crosslinking of the extracellular matrix component and the like may also include physical crosslinking by application of heat, ultraviolet rays, radiation, or the like, and crosslinking by chemical crosslinking by a crosslinking agent, an enzyme reaction, or the like, for example.

The type of ions of a metal element contained in the gel composition according to the present embodiment is not particularly limited, and ions of any metal element can be used.

From the viewpoint that the effect of the present invention that the gel composition is transparent and the elastic modulus of the gel composition is high (meaning hard) can be exhibited more significantly, the metal element is preferably at least one metal element selected from the group consisting of transition metal elements and base metal elements. Specific examples of the at least one metal element selected from the group consisting of transition metal elements and base metal elements include titanium (Ti), copper (Cu), zinc (Zn), palladium (Pd), platinum (Pt), and gold (Au). Specific examples of ions of the at least one metal element selected from the group consisting of transition metal elements and base metal elements include Ti⁺, Ti²⁺, Ti³⁺, Ti⁴⁺, Cu⁺, Cu²⁺, Zn⁺, Zn²⁺, Pd²⁺, Pd⁴⁺, Pt²⁺, Pt⁴⁺, Au⁺, Au²⁺, and Au⁴⁺.

From the viewpoint that the effect of the present invention described above can be exhibited more significantly, the metal element is more preferably at least one metal element selected from the group consisting of transition metals of Group 10, transition metals of Group 11, and metals of Group 12 of the periodic table. Specific examples of the at least one metal element selected from the group consisting of transition metals of Group 10, transition metals of Group 11, and metals of Group 12 of the periodic table include copper (Cu), Zinc (Zn), Palladium (Pd), Platinum (Pt), and Gold (Au). Specific examples of ions of the at least one metal element selected from the group consisting of transition metals of Group 10, transition metals of Group 11, and metals of Group 12 of the periodic table include Cu²⁺, Zn²⁺, Pd²⁺, Pd⁴⁺, Pt²⁺, Pt⁴⁺, Au⁺, Au²⁺, and Au⁴⁺.

The ions of the metal element used in the gel composition according to the present embodiment are preferably ions of a metal element selected from titanium (Ti), copper (Cu), zinc (Zn), platinum (Pt), and gold (Au) from the viewpoint of no cytotoxicity or low cytotoxicity, and safety for living organisms. When the gel composition is safe for living organisms, the gel composition can be suitably used as a scaffold material when culturing cells at the time of forming an artificial tissue, for example.

Since the gel composition according to the present embodiment is transparent, inside thereof can easily observed. Accordingly, the gel composition can be suitably used as a scaffold material when culturing cells at the time of forming an artificial tissue, for example. The total light transmittance of the gel composition according to the present embodiment may be 80% or more, may be 85% or more, may be 90% or more, or may be 95% or more, for example. The total light transmittance is defined as the ratio of the transmitted luminous flux (including the diffuse component) to the parallel incident luminous flux of a prepared test piece composed of a gel composition having the thickness of 10 mm.

Since the gel composition according to the present embodiment has a high elastic modulus, it can be suitably used as a scaffold material when culturing cells at the time of artificially forming a cancer tissue, for example. Accordingly, an artificial cancer tissue having a high elastic modulus (meaning hard) can be formed. The elastic modulus of the gel composition according to the present embodiment may be 1.5 kPa or more, may be 2.0 kPa or more, may be 5.0 kPa or more, may be 10 kPa or more, or may be 50 kPa or more. Although the upper limit of the elastic modulus is not particularly limited, it is usually 250 kPa or less, preferably 200 kPa or less, and more preferably 150 kPa or less. The elastic modulus is a value measured by a method described in Examples to be described later.

The content of the at least one selected from the group consisting of the extracellular matrix component and the fragmented extracellular matrix component in the gel composition according to the present embodiment may be 0.01% by weight or more, may be 0.05% by weight or more, may be 0.1% by weight or more, may be 0.15% by weight or more, may be 0.2% by weight or more, may be 0.5% by weight or more, may be 1.0% by weight or more, may be 2.0% by mass or more, or may be 3.0% by mass or more, for example, based on the total amount of the gel composition. The upper limit of the content of the at least one selected from the group consisting of the extracellular matrix component and the fragmented extracellular matrix component is not particularly limited as long as preparation possible, but for example, it may be 10.0% by weight or less, or may be 5.0% by weight or less based on the total amount of the gel composition.

The content of the ions of the metal element in the gel composition according to the present embodiment may be 5 mg or more, may be 10 mg or more, may be 20 mg or more, may be 30 mg or more, or may be 40 mg or more, for example, per 1 g of the total content of the at least one selected from the group consisting of the extracellular matrix component and the fragmented extracellular matrix component. The content of the ions of the metal element is not particularly limited, but it may be 100 mg or less, may be 80 mg or less, or may be 60 mg or less, for example, per 1 g of the total content of the at least one selected from the group consisting of the extracellular matrix component and the fragmented extracellular matrix component.

Depending on the usage of the gel composition, the gel composition according to the present embodiment may further contain another component other than the at least one selected from the group consisting of the extracellular matrix component and the fragmented extracellular matrix component, and the ions of the metal element. When the gel composition is used as a scaffold material when culturing cells, examples of the other component include a nutritional component for cells to be cultured and a pH adjuster.

### [Production method for gel composition]

The gel composition according to the present embodiment can be obtained by, for example, a production method including a step (contacting step) of bringing a solution containing the at least one selected from the group consisting of the extracellular matrix component and the fragmented extracellular matrix component into contact with a solution containing the ions of the metal element.

The solution containing the at least one selected from the group consisting of the extracellular matrix component and the fragmented extracellular matrix component can be obtained by dissolving or dispersing the above-described extracellular matrix component and/or fragmented extracellular matrix component in a solvent. The solvent is not particularly limited as long as it can dissolve or disperse the extracellular matrix component and/or the fragmented extracellular matrix component, but specific examples thereof include water, physiological saline such as phosphate buffered saline (PBS), and liquid media such as a Dulbecco's Modified Eagle medium (DMEM).

The solution containing the ions of the metal element is not particularly limited as long as it contains the ions of the above-mentioned metal element. The ion source of the ions of the metal element is also not particularly limited, and may be an inorganic salt, an organic salt, or the like, for example. The solution containing the ions of the metal element can be obtained by dissolving the ion source of the ions of the metal element in a solvent, for example. The solvent is not particularly limited as long as it can dissolve the ion source of the ions of the metal element, and may be water, organic solvents such as ethanol and dimethyl sulfoxide (DMSO), or the like, for example.

For the contacting step, any method can be adopted as long as it is a method of bringing the solution containing the at least one selected from the group consisting of the extracellular matrix component and the fragmented extracellular matrix component into contact with the solution containing the ions of the metal element. Specific examples thereof include a method of bringing the solution containing the at least one selected from the group consisting of the extracellular matrix component and the fragmented extracellular matrix component into contact with the solution containing the ions of the metal element by mixing. When mixing, it is preferable to mix both solutions so that they are homogeneous. In addition, from the viewpoint of further increasing the formation efficiency of a gel, it is preferable to bring both solutions into contact with each other by a method in which, while stirring the solution containing the at least one selected from the group consisting of the extracellular matrix component and the fragmented extracellular matrix component, the solution containing the ions of the metal element is added dropwise thereto. The dropwise addition rate can be 1 drop/second, for example.

The temperature when implementing the contacting step is not particularly limited, but from the viewpoint of avoiding an excessive reaction, the implementation at 10°C or lower is preferable, and the implementation at 5°C or lower is more preferable, for example. Although the lower limit of the temperature is not particularly limited, it is usually -3°C or higher, and is preferably 0°C or higher.

The contact time of both solutions when implementing the contacting step is appropriately set such that gel formation occurs, according to the type of the extracellular matrix component and/or the fragmented extracellular matrix component used and the type of the ions of the metal element. The contact time is usually 0 seconds or longer (gelation immediately after contact) and 30 minutes or shorter. For example, when a fragmented collagen solution and a Pd²⁺ solution are brought into contact with each other, the contact time of 0 seconds or longer and 10 seconds or shorter is sufficient because gelation occurs instantaneously upon contact. In addition, for example, when a collagen solution and a Pt⁴⁺ solution are brought into contact with each other, a gel can be formed by setting the contact time to 10 minutes or longer and 20 minutes or shorter.

### [Use method for gel composition]

Since the gel composition according to the present embodiment is transparent and has a high elastic modulus (meaning hard), it is suitably used as a scaffold material for forming a cell structure (three-dimensional tissue body), for example.

### [Three-dimensional tissue body]

The three-dimensional tissue body is a cell agglomerate in which cells are three-dimensionally disposed via the extracellular matrix component, and is an agglomerate artificially produced by cell culture. The shape of the three-dimensional tissue body is not particularly limited, and examples thereof include sheet, spherical, ellipsoidal, and rectangular parallelepiped shapes.

The three-dimensional tissue body according to the present embodiment contains at least the at least one selected from the group consisting of the extracellular matrix component and the fragmented extracellular matrix component, cells, and the ions of the metal element. At least some of the cells may be in contact with the extracellular matrix component and/or the fragmented extracellular matrix component. Adhesion may be one aspect of contact.

The three-dimensional tissue body according to the present embodiment may be transparent at 37°C, for example. Specifically, the total light transmittance at 37°C may be 80% or more, may be 85% or more, may be 90% or more, or may be 95% or more, for example. The total light transmittance is defined according to the total light transmittance of the gel composition.

The cells contained in the three-dimensional tissue body according to the present embodiment are not particularly limited, but may be cells derived from animals such as humans, monkeys, dogs, cats, rabbits, pigs, cows, mice, and rats, for example. The site from which the cells are derived is not particularly limited, and may be somatic cells derived from bones, muscles, internal organs, nerves, brains, bones, skin, blood, or the like, or may be germ cells. Furthermore, the cells may be induced pluripotent stem cells (iPS cells) or embryonic stem cells (ES cells), or may be cultured cells such as primary cultured cells, subcultured cells, and cell line cells. Specific examples of the cells include, but are not limited to nerve cells, dendritic cells, immune cells, vascular endothelial cells (for example, human umbilical vein endothelial cells (HUVEC)), lymphatic endothelial cells, fibroblasts, cancer cells such as colon cancer cells (for example, human colon cancer cells (HT29)) and liver cancer cells, epithelial cells (for example, human gingival epithelial cells), keratinocytes, cardiomyocytes (for example, human iPS cell-derived cardiomyocytes (iPS-CM)), hepatocytes, pancreatic islet cells, tissue stem cells, and smooth muscle cells (for example, aortic smooth muscle cells (Aorta-SMC)). For the cells, one type of cell may be used alone, or multiple types of cells may be used in combination.

Specific aspects of the extracellular matrix component and the fragmented extracellular matrix component, and the ions of the metal element contained in the three-dimensional tissue body according to the present embodiment are the same as the aspect described for the gel composition of the present invention.

The thickness of the three-dimensional tissue body according to the present embodiment is preferably 10 µm or more, more preferably 100 µm or more, and further more preferably 1000 µm or more. Such a three-dimensional tissue body has a structure closer to that of a biological tissue, and is suitable as a substitute for experimental animals, and a grafting material. The upper limit of the thickness of the three-dimensional tissue body according to the present embodiment is not particularly limited, but may be 10 mm or less, may be 3 mm or less, may be 2 mm or less, may be 1.5 mm or less, or may be 1 mm or less, for example. The thickness of the three-dimensional tissue body means the distance between both ends in the direction perpendicular to a main surface when the three-dimensional tissue body has a sheet shape or a rectangular parallelepiped shape. When the main surface has unevenness, the thickness means the distance at the thinnest part of the above-mentioned main surface. In addition, when the three-dimensional tissue body has a spherical shape, the thickness means the diameter thereof. Furthermore, when the three-dimensional tissue body has an ellipsoidal shape, the thickness means the minor axis thereof. When the three-dimensional tissue body has a substantially spherical shape or a substantially ellipsoidal shape and has unevenness on the surface, the thickness means the distance between two points where a straight line passing through the center of gravity of the three-dimensional tissue body and the above-mentioned surface intersects, and means the shortest distance.

### [Production method for three-dimensional tissue body]

The three-dimensional tissue body according to the present embodiment can be obtained by a production method including, for example: a step (contacting step) of obtaining a cell-containing gel composition by bringing at least one (hereinafter also referred to as "extracellular matrix component and the like") selected from the group consisting of an extracellular matrix component and a fragmented extracellular matrix component, cells, and ions of a metal element into contact with each other in an aqueous medium; and a step (culturing step) of culturing the cell-containing gel composition obtained in the contacting step. The production method according to the present embodiment may further include a step (incubation step) of incubating the cell-containing gel composition at 20°C to 30°C after the contacting step and before the culturing step.

The contacting step is a step of obtaining the cell-containing gel composition by bringing the extracellular matrix component and the like, the cells, and the ions of the metal element into contact with each other in the aqueous medium. The aqueous medium means a liquid having water as an essential constituent component. Examples of the aqueous medium include physiological saline such as phosphate buffered saline (PBS), and liquid media such as a Dulbecco's Modified Eagle medium (DMEM). The liquid medium may be a mixed medium in which two types of media have been mixed. The aqueous medium is preferably a liquid medium from the viewpoint of reducing the load on the cells.

The liquid medium is not particularly limited, and a suitable medium can be selected according to the type of cells to be cultured. Examples of the medium include an Eagle's MEM medium, a DMEM, a Modified Eagle medium (MEM), a Minimum Essential medium, an RPMI medium, and a GlutaMax medium. The medium may be a serum-supplemented medium or may be a serum-free medium. Furthermore, the liquid medium may be a mixed medium in which two or more types of media have been mixed.

Specific aspects of the extracellular matrix component and the like and the ions of the metal element, which are used in the contacting step, are the same as those described for the gel composition according to the present invention.

The contacting step may include, for example, bringing a suspension containing the extracellular matrix component and the like, the cells, and a first aqueous medium into contact with a solution containing the ions of the metal element and a second aqueous medium. In this case, the first aqueous medium and the second aqueous medium may be the same as or different from each other. The above-mentioned suspension can be obtained by mixing a solution in which the extracellular matrix component and the like have been dissolved in the first aqueous medium with the cells, for example.

The concentration of the extracellular matrix component and the like in the contacting step can be appropriately determined according to the shape and thickness of the target three-dimensional tissue body, the size of an incubator, and the like. For example, the concentration of the extracellular matrix component and the like in the aqueous medium in the contacting step may be 0.1% to 30% by mass, or may be 0.1% to 10% by mass based on the total amount of the aqueous medium (for example, the total amount of the first aqueous medium and the second aqueous medium). Furthermore, the amount of the extracellular matrix component and the like with respect to 1 × 10⁶ cells may be 0.01 to 1000 µg, may be 0.1 to 100 µg, or may be 0.1 to 5 µg, for example.

The culturing step is a step of culturing the cell-containing gel composition obtained in the contacting step. The culturing step is preferably performed under the condition in which at least some of the cells contained in the cell-containing gel composition maintain a viable state. The condition for maintaining the viable state can be appropriately set according to the type of cells. Specific examples thereof include culture conditions exemplified below.

Regarding the culture conditions in the culturing step, suitable culture conditions can be set according to the type of cells. For example, the culture temperature may be 20°C to 40°C, or may be 30°C to 37°C. The pH of the medium may be 6 to 8, or may be 7.2 to 7.4. The culture time may be 1 day to 14 days, may be 7 days to 14 days, may be 14 days to 30 days, may be 30 days to 60 days, or may be 60 days to 90 days.

The cell density in the cell-containing gel composition can be appropriately determined according to the shape, thickness, and the like of the target three-dimensional tissue body. For example, the cell density in the cell-containing gel composition may be 1 to 10⁸ cells/ml, or may be 10³ to 10⁷ cells/ml.

The incubation step is a step of incubating the cell-containing gel composition at 20°C to 30°C after the contacting step and before the culturing step. The incubation step is a step that is implemented to more reliably form a gel with the extracellular matrix component and the like and the ions of the metal element. The incubation step may be implemented as necessary. The incubation time can be appropriately set depending on the types of the extracellular matrix component and the like and the ions of the metal element used, and the like, but the time can be 30 minutes to 24 hours, for example.

### Example

Hereinafter, the present invention will be described more specifically based on test examples. However, the present invention is not limited to the following test examples.

### [Test Example 1: Production of gel composition]

### (Preparation of collagen solution)

1 g of porcine skin-derived type I and type III mixed collagen manufactured by NH Foods Ltd. was added to 500 mL of ultrapure water, and incubated at 4°C for 12 hours to produce a 0.2% by weight collagen solution. Thereafter, 0.45M NaCl and 5mM Tris-HCl were added to the collagen solution to be incubated at 4°C for 12 hours, and thereafter 1.2M NaCl was added to be further incubated at 4°C for 12 hours. After the incubation, the mixture was centrifuged at 10000 rpm for 15 minutes, and the supernatant was recovered to obtain a purified type I collagen solution. The obtained type I collagen solution was dialyzed against ultrapure water for 7 days (molecular weight cut-off (MWCO): 15 kDa), and thereafter freeze-dried for 3 days to obtain purified type I collagen.

The obtained purified type I collagen was added to PBS (pH 7) to be incubated at 4°C for 12 hours, thereby preparing a 0.2% by weight type I collagen solution.

### (Preparation of Au³⁺ solution)

An aqueous solution of sodium hydroxide (NaOH) or water was added to an aqueous solution of tetrachloridogold(III) acid (HAuCl₄) to respectively obtain a 12.5 mM Au³⁺ solution (pH 6.8) or a 12.5 mM Au³⁺ solution (pH 2.2). As a control, hydrochloric acid (HCl aqueous solution, pH 2.2) was prepared.

### (Preparation of gel composition)

### <Example 1>

6 mL of the 0.2% by weight type I collagen solution was put in a container to which a stirrer bar had been put, and was stirred while cooling in an ice bath. Subsequently, while stirring the collagen solution in the ice bath, 240 µL of the Au³⁺ solution (pH 6.8) was added dropwise thereto (Au³⁺: 3 µmol).

### <Example 2>

The same operation as in Example 1 was performed except that 240 µL of the Au³⁺ solution (pH 2.2) was added dropwise (Au³⁺: 3 µmol) instead of the Au³⁺ solution (pH 6.8).

### <Comparative Example 1>

The same operation as in Example 1 was performed except that 240 µL of the hydrochloric acid (pH 2.2) was added dropwise instead of the Au³⁺ solution (pH 6.8).

### (Measurement of elastic modulus)

The elastic modulus of the gel was measured using a small tabletop tester EZ-test (manufactured by Shimadzu Corporation). Specifically, the gel was compressed at the temperature of 25°C and the compression rate of 1 mm/min with a rod-shaped jig (tip area: 11 mm²) set in a load cell of the tester to obtain a stress-strain line (Stress-Strain curve). The Young's modulus (kPa) was calculated from the slope of an elastic deformation region at the initial stage of stress rise in the obtained stress-strain line, and was denoted by the elastic modulus.

### (Results)

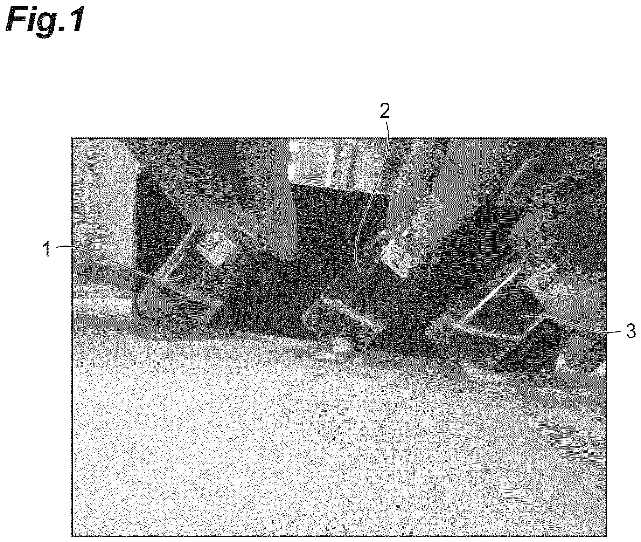

FIG. 1 is a photograph showing the appearance of each composition when 10 minutes had elapsed after dropwise addition of the Au³⁺ solution or hydrochloric acid. In FIG. 1, a container 1 indicates a composition obtained by dropwise addition of the Au³⁺ solution (pH 6.8), a container 2 indicates a composition obtained by dropwise addition of the Au³⁺ solution (pH 2.2), and a container 3 indicates a composition obtained by dropwise addition of hydrochloric acid (pH 2.2).

As shown in FIG. 1, the composition of the container 1, which was obtained by dropwise addition of the Au³⁺ solution (pH 6.8) onto the type I collagen, became a transparent gel (elastic modulus 2.2 kPa). Similarly, the composition of the container 2, which was obtained by dropwise addition of the Au³⁺ solution (pH 2.2) onto the type I collagen, became a transparent gel (elastic modulus 2.2 kPa). On the other hand, the composition of the container 3, which was obtained by dropwise addition of the hydrochloric acid (pH 2.2) onto the type I collagen, was not gelated. From these results, it was found that gelation of collagen requires the presence of metal ions (Au³⁺) rather than pH.

### [Test Example 2: Production of gel composition]

### (Preparation of collagen solution)

A 0.2% by weight type I collagen solution was produced in the same manner as in Test Example 1. In addition, 1 g of porcine skin-derived type I and type III mixed collagen manufactured by NH Foods Ltd. was added to 500 mL of ultrapure water, and incubated at 4°C for 12 hours to produce a 0.2% by weight type I and type III mixed collagen solution.

### (Preparation of Au³⁺ solution)

12.5 mM of the Au³⁺ solution (pH 6.8) was prepared in the same manner as in Test Example 1.

### (Preparation of gel composition)

### <Example 3>

6 mL of the 0.2% by weight type I collagen solution was put in a container to which a stirrer bar had been put, and was stirred while cooling in an ice bath. Subsequently, while stirring the collagen solution in the ice bath, 240 µL of the Au³⁺ solution (pH 6.8) was added dropwise thereto (Au³⁺: 3 µmol).

### <Example 4>

6 mL of the 0.2% by weight type I and type III mixed collagen solution was put in a container to which a stirrer bar had been put, and was stirred while cooling in an ice bath. Subsequently, while stirring the collagen solution in the ice bath, 240 µL of the Au³⁺ solution (pH 6.8) was added dropwise thereto (Au³⁺: 3 µmol).

### (Results)

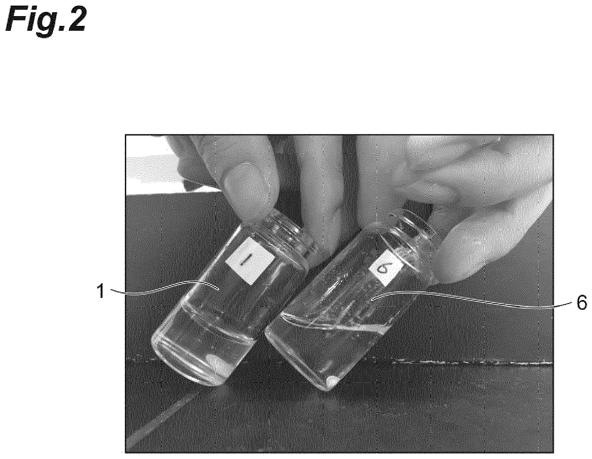

FIG. 2 is a photograph showing the appearance of each composition when 10 minutes had elapsed after dropwise addition of the Au³⁺ solution. In FIG. 2, a container 1 indicates a composition obtained by dropwise addition of the Au³⁺ solution (pH 6.8) onto the type I collagen solution, and a container 6 indicates a composition obtained by dropwise addition of the Au³⁺ solution (pH 6.8) onto the type I and type III mixed collagen solution.

As shown in FIG. 2, the composition of the container 1, in which the Au³⁺ solution (pH 6.8) was added dropwise onto the type I collagen solution, was a transparent gel (elastic modulus 2.2 kPa). The composition in container 6, which was obtained by dropwise addition of the Au³⁺ solution (pH 6.8) onto the type I and type III mixed collagen solution, also became a transparent gel, but this was a gel softer than the gel composition of the container 1.

### [Test Example 3: Production of gel composition]

### (Preparation of collagen solution)

A 0.2% by weight type I collagen solution was prepared in the same manner as in Test Example 1.

### (Preparation of fragmented collagen solution)

50 mg of purified type I collagen obtained by the same operation as in Test Example 1 was suspended in 5 mL of 1 × PBS (pH = 7), and homogenization was performed at room temperature for 6 minutes using a stirring homogenizer. Thereafter, incubation was further performed at 4°C for 3 days to obtain a 1% by weight fragmented collagen solution. The average diameter of the fragmented collagen in the obtained fragmented collagen solution was 84.4 ± 43.0 nm (N = 25).

### (Preparation of Pd²⁺ solution)

Palladium chloride (H₂PdCl₄) was dissolved in water to prepare a 12.5 mM Pd²⁺ solution (pH 1.1).

### (Preparation of gel composition)

### <Example 5>

6 mL of the 0.2% by weight type I collagen solution was put in a container to which a stirrer bar had been put, and was stirred while cooling in an ice bath. Subsequently, while stirring the collagen solution in the ice bath, 240 µL of the Pd²⁺ solution was added dropwise thereto (Pd²⁺: 3 µmol).

### <Example 6>

6 mL of the 1% by weight fragmented collagen solution was put in a container to which a stirrer bar had been put, and was stirred while cooling in an ice bath. Subsequently, while stirring the fragmented collagen solution in the ice bath, 240 µL of the Pd³⁺ solution was added dropwise thereto (Pd³⁺: 3 µmol).

### (Results)

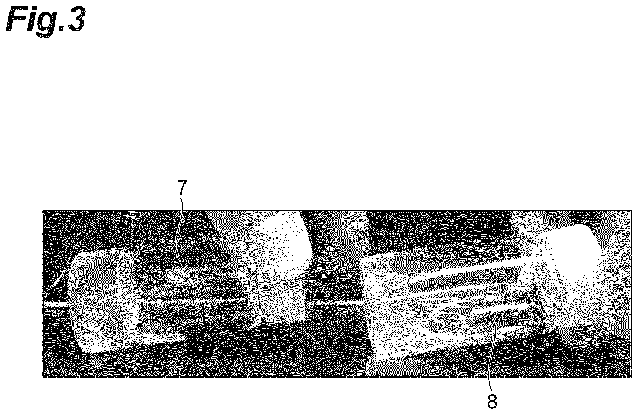

FIG. 3 is a photograph showing the appearance of each composition when 10 minutes had elapsed after dropwise addition of the Pd²⁺ solution. In FIG. 3, a container 7 indicates a composition obtained by dropwise addition of the Pd²⁺ solution onto the type I collagen solution, and a container 8 indicates a composition obtained by dropwise addition of the Pd²⁺ solution onto the fragmented collagen solution.

As shown in FIG. 3, the composition of the container 7, which was obtained by dropwise addition of the Pd²⁺ solution onto the type I collagen solution, became a transparent gel (elastic modulus 2.4 kPa), although coloration due to Pd²⁺ was recognized. The composition of the container 8, which was obtained by dropwise addition of the Pd²⁺ solution onto the fragmented collagen solution, also became a transparent gel (elastic modulus 116 kPa), although coloration due to Pd²⁺ was recognized. In particular, in the composition of the container 8, the portion onto which the Pd²⁺ solution was added dropwise was instantly gelated, thereby forming a harder gel.

### [Test Example 4: Production of gel composition]

### (Preparation of collagen solution)

A 0.2% by weight type I collagen solution was prepared in the same manner as in Test Example 1.

### (Preparation of Au⁺ solution)

Chloro[(tetrahydrothiophen-1-ium)-1-yl]gold(III) was dissolved in dimethyl sulfoxide (DMSO) to prepare a 12.5 mM Au⁺ solution.

### (Preparation of gel composition)

### <Example 7>

6 mL of the 0.2% by weight type I collagen solution was put in a container to which a stirrer bar had been put, and was stirred while cooling in an ice bath. Subsequently, while stirring the collagen solution in the ice bath, 240 µL of the Au⁺ solution was added dropwise thereto (Au⁺: 3 µmol).

### <Comparative Example 2>

6 mL of the 0.2% by weight type I collagen solution was put in a container to which a stirrer bar had been put, and was stirred while cooling in an ice bath. Subsequently, while stirring the collagen solution in the ice bath, DMSO was added dropwise thereto.

### <Comparative Example 3>

6 mL of the 0.2% by weight type I collagen solution was put in a container to which a stirrer bar had been put, and was stirred while cooling in an ice bath. Subsequently, while stirring the collagen solution in the ice bath, a chloro[(tetrahydrothiophen-1-ium)-1-yl]gold(III) powder was added thereto. Stirring was continued even after the addition, but the powder did not dissolve in the solution.

### (Results)

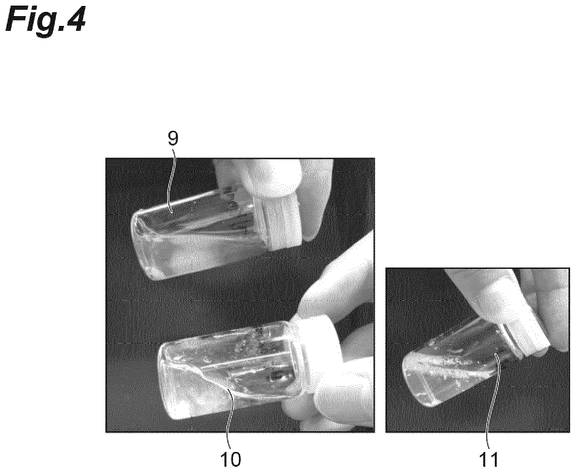

FIG. 4 shows photographs showing the appearance of each composition when 10 minutes had elapsed after dropwise addition or addition of the Au⁺ solution, DMSO, or chloro[(tetrahydrothiophen-1-ium)-1-yl]gold(III) powder. In FIG. 4, a container 9 indicates a composition obtained by dropwise addition of DMSO onto the type I collagen solution, a container 10 indicates a composition obtained by dropwise addition of the Au⁺ solution onto the type I collagen solution, and a container 11 indicates a composition obtained by addition of the chloro[(tetrahydrothiophen-1-ium)-1-yl]gold(III) powder to the type I collagen solution.

As shown in FIG. 4, in the composition of the container 9 which was obtained by dropwise addition of DMSO onto the type I collagen solution, gelation of the entire solution was not confirmed, although gelation was locally recognized at the dropwise addition portion. The composition of the container 10, which was obtained by dropwise addition of the Au⁺ solution onto the type I collagen solution, became a transparent gel. On the other hand, in the composition of the container 11 which was obtained by adding the chloro[(tetrahydrothiophen-1-ium)-1-yl]gold(III) powder to the type I collagen solution, the powder did not dissolve in the solution.

### [Test Example 5: Production of gel composition]

### (Preparation of collagen solution)

A 1% by weight fragmented collagen solution was prepared in the same manner as in Test Example 3.

### (Preparation of Pt⁴⁺ solution)

Hexachloroplatinic acid (H₂PtCl₆) was dissolved in water to prepare a 12.5M Pt⁴⁺ solution.

### (Preparation of gel composition)

### <Example 8>

6 mL of the 1% by weight fragmented collagen solution was put in a container to which a stirrer bar had been put, and was stirred while cooling in an ice bath. Subsequently, the fragmented collagen solution was left to stand in the ice bath, and 240 µL of the Pt⁴⁺ solution was added dropwise thereto (Pt⁴⁺: 3 µmol).

### (Results)

In the composition obtained by dropwise addition of the Pt⁴⁺ solution onto the type I collagen solution, gelation was not recognized immediately after the dropwise addition, but the composition became a transparent gel (elastic modulus 89 kPa) after being left to stand in the ice bath for 15 minutes.

### [Test Example 6: Production and evaluation of three-dimensional tissue body]

### (Preparation of collagen solution)

A 0.2% by weight type I collagen solution was prepared in the same manner as in Test Example 1.

### (Preparation of fragmented collagen solution)

50 mg of purified type I collagen obtained by the same operation as in Test Example 1 was suspended in 5 mL of 1 × PBS (pH = 7), and homogenization was performed at room temperature for 6 minutes using a stirring homogenizer. Thereafter, incubation was further performed at 4°C for 3 days to obtain a 1.0% by weight fragmented collagen solution. Furthermore, the above-mentioned fragmented collagen solution was diluted with an RPMI-1640 medium to obtain a 0.5% by weight fragmented collagen solution.

### (Preparation of Pt²⁺ solution)

Potassium tetrachloroplatinate(II) (K₂PtCl₄) was dissolved in PBS to prepare a 12.5 mM Pt²⁺ solution.

### (Preparation of cell suspension)

1.0 × 10⁶ cells of human mammary gland cancer cells (MDA-MB-231) was suspended in 64 µL of an RPMI-1640 medium containing 10% FBS, and the total amount was seeded in an insert of a 24-well insert (CORNING 3470).

### (Preparation and evaluation of three-dimensional tissue body)

### <Example 9>

To the insert of the 24-well insert in which the cells had been seeded, 224 µL of the 0.5% by weight fragmented collagen solution and 12 µL of the 12.5 mM Pt²⁺ solution were further added and suspended, and incubation was performed at room temperature for 30 minutes to obtain a cell-containing gel composition. Thereafter, 2 mL of the RPMI-1640 medium was added to the outside of the insert and cultured for 4 days by performing medium exchange every other day, thereby preparing a three-dimensional tissue body. When culturing was performed for 4 days (day 4), the appearance and the inside of the three-dimensional tissue body were observed using a phase-contrast microscope (CKX53 manufactured by Olympus Corporation).

### (Results)

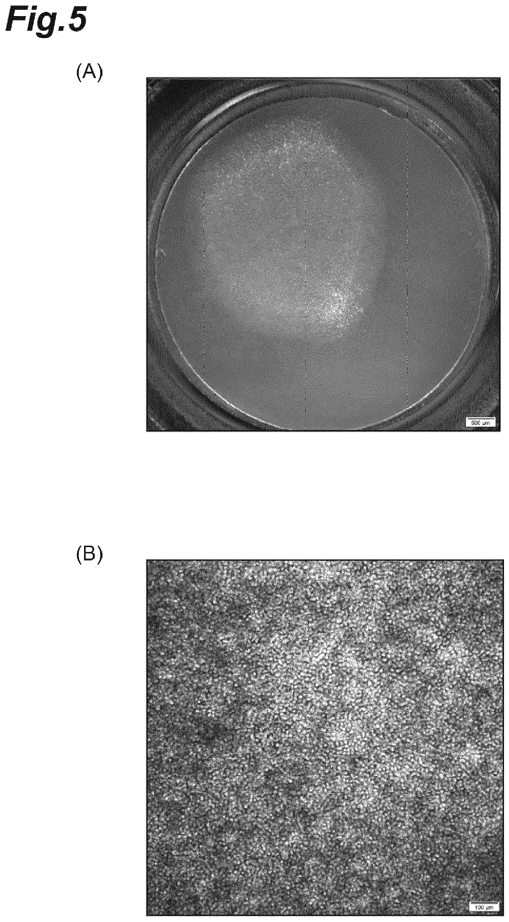

FIG. 5(A) is a photograph showing the appearance of the three-dimensional tissue body. FIG. 5(B) is a photograph showing the inside of the three-dimensional tissue body. As shown in FIG. 5(A) and FIG. 5(B), the three-dimensional tissue body obtained in Example 9 had a transparent appearance, which made observation of the cells inside possible, although coloration due to the medium components and the like was recognized. When the cell suspension and the Pt²⁺ solution alone were mixed to culture the cells and confirm the viability of the cells, the viability was 81% to 87% (concentration dependency was not recognized) under conditions in which the final concentration of Pt²⁺ was 0.05 mM to 0.5 mM, and significant cytotoxicity was not recognized.

### [Test Example 7: Production of gel composition]

### (Preparation of fragmented collagen solution)

A 1% by weight fragmented collagen solution was prepared in the same manner as in Test Example 3.

### (Preparation of Pt²⁺ solution)

Potassium tetrachloroplatinate(II) (K₂PtCl₄) was dissolved in PBS to respectively prepare 25 mM, 12.5 mM, 2.5 mM, 1.25 mM, 0.25 mM, and 0.125 mM Pt²⁺ solutions.

### (Preparation of Au³⁺ solution)

Tetrachloridogold(III) acid (HAuCl₄) was dissolved in PBS to respectively prepare 25 mM, 12.5 mM, 2.5 mM, 1.25 mM, 0.25 mM, and 0.125 mM Au³⁺ solutions.

### (Preparation of gel composition)

### <Example 10>

To the insert of a 24-well insert, 288 µL of the 1% by weight fragmented collagen solution and 12 µL of the Pt²⁺ solution or Au³⁺ solution at each concentration were further added and suspended, and incubation was performed at room temperature for 30 minutes to obtain a gel composition. In addition, the elastic modulus was evaluated after incubation at 37°C for 24 hours. The measurement of the elastic modulus was performed in the same procedure as in Test Example 1.

### (Results)

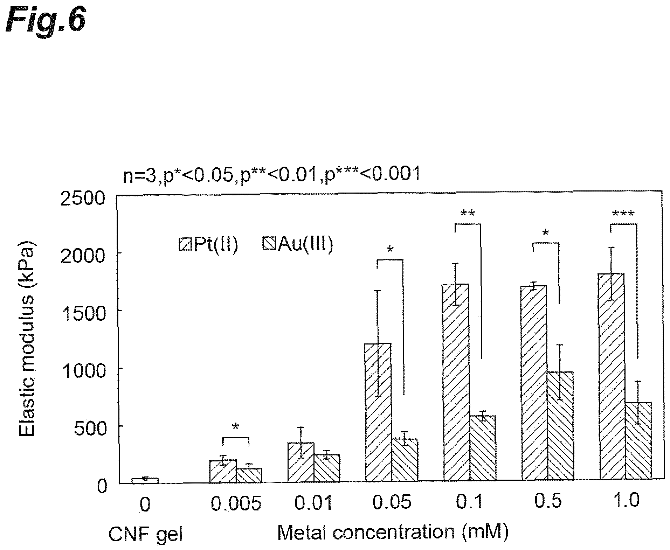

FIG. 6 is a graph showing the elastic modulus of the gel composition produced from the solution of each metal ion. The horizontal axis indicates the final concentration of each metal ion. As shown in FIG. 6, the elastic modulus of the gel was improved as the concentration of the metal ions increased. In addition, the case of using the Pt²⁺ solution enabled the production of the gel having a higher elastic modulus as compared to the case of using the Au³⁺ solution. Regardless of which metal ion solution was used, the elastic modulus of the gel increased in a manner dependent on the concentration of the metal ions until the final concentration reached 0.1 mM, and an approximately constant elastic modulus was shown in the concentration range after 0.1 mM.

### [Test Example 8: Production of gel composition]

### (Preparation of fragmented collagen solution)

A 1% by weight fragmented collagen solution was prepared in the same manner as in Test Example 3. Subsequently, the prepared fragmented collagen solution was diluted with PBS to prepare 0.2% by weight and 0.5% by weight fragmented collagen solutions.

### (Preparation of Pt²⁺ solution)

Potassium tetrachloroplatinate(II) (K₂PtCl₄) was dissolved in PBS to respectively prepare 12.5 mM, 2.5 mM, 1.25 mM, 0.25 mM, and 0.125 mM Pt²⁺ solutions.

### (Preparation of gel composition)

### <Example 11>

To the wells of a 96-well microplate, 288 µL of the 1% by weight fragmented collagen solution at each concentration and 12 µL of the Pt²⁺ solution at each concentration were further added and suspended, and incubation was performed at room temperature for 30 minutes to obtain a gel composition. Furthermore, after incubation at 37°C for 24 hours, the absorbance at the wavelength of 500 nm was measured to calculate the transmittance. Furthermore, the measurement of the elastic modulus was performed in the same procedure as in Example 1.

### (Results)

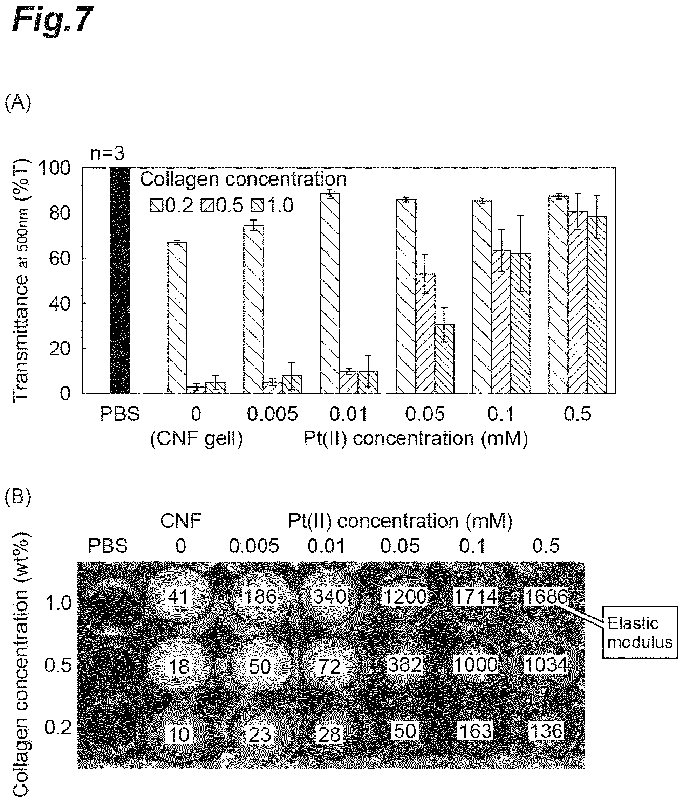

FIG. 7(A) is a graph showing the transmittance at the wavelength of 500 nm of gel compositions produced from a fragmented collagen solution and a Pt²⁺ solution at each concentration. The horizontal axis indicates the final concentration of metal ions (Pt²⁺). As shown in FIG. 7(A), the transmittance of the gel was improved as the concentration of the metal ions increased. In particular, when the final concentration of the metal ions was 0.05 mM or more, the transmittance was significantly improved as compared to when the final concentration was less than 0.05 mM. FIG. 7(B) is an image observed from the top surface of gels produced from the fragmented collagen solution and the Pt²⁺ solution at each concentration. In FIG. 7(B), the numerical value at the center of each well indicates the elastic modulus of the gel composition in the corresponding well. The interesting results were observed that the transmittance was improved despite the improvement of the elastic modulus.

### [Test Example 9: Production of gel composition]

### (Preparation of fragmented collagen solution)

A 0.5% by weight fragmented collagen solution was prepared in the same manner as in Test Example 6.

### (Preparation of Pd²⁺ solution)

Palladium(II) chloride (PdCl₂) was dissolved in water to prepare a 12.5 mM Pd²⁺ solution.

### (Preparation of Cu²⁺ solution)

Copper (II) chloride (CuCl₂) was dissolved in water to prepare a 12.5 mM Cu²⁺ solution.

### (Preparation of Zn²⁺ solution)

Zinc(II) chloride (ZnCl₂) was dissolved in water to prepare a 12.5 mM Zn²⁺ solution.

### (Preparation of gel composition)

### <Example 12>

To a glass container, 480 µL of the 0.5% by weight fragmented collagen solution and 20 µL of each metal ion solution were further added and suspended, and incubation was performed at room temperature for 30 minutes to obtain a gel composition. In addition, the appearance was evaluated after incubation at 4°C for 24 hours.

### (Results)

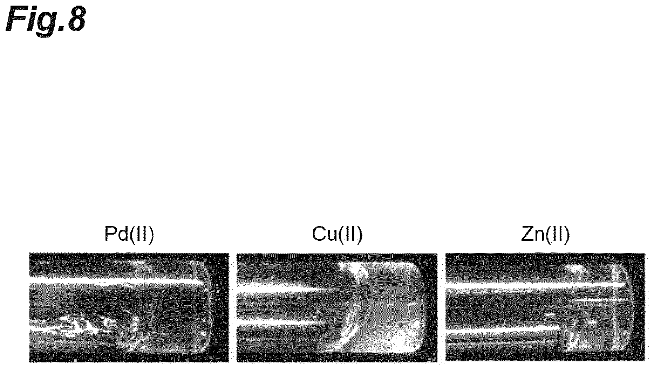

FIG. 8 shows photographs showing the appearance of each composition after 1 day had elapsed after adding each metal solution. The compositions produced using any of the metal ions became transparent gels.

## Claims

1. A gel composition comprising:
at least one selected from the group consisting of an extracellular matrix component and a fragmented extracellular matrix component; and
an ion of a metal element.

2. The gel composition according to claim 1, wherein the metal element is at least one metal element selected from the group consisting of transition metal elements and base metal elements.

3. The gel composition according to claim 1 or 2, wherein the metal element is at least one metal element selected from the group consisting of transition metals of Group 10, transition metals of Group 11, and metals of Group 12 of the periodic table.

4. The gel composition according to any one of claims 1 to 3, wherein the metal element is at least one selected from the group consisting of copper, zinc, palladium, platinum, and gold.

5. The gel composition according to any one of claims 1 to 4, wherein the extracellular matrix component contains collagen.

6. The gel composition according to any one of claims 1 to 5, wherein the fragmented extracellular matrix component contains fragmented collagen.

7. The gel composition according to any one of claims 1 to 6, wherein at least one selected from the group consisting of the extracellular matrix component and the fragmented extracellular matrix component is crosslinked.

8. A production method for a gel composition, the method comprising a step of bringing a solution containing at least one selected from the group consisting of an extracellular matrix component and a fragmented extracellular matrix component into contact with a solution containing an ion of a metal element.

9. A production method for a three-dimensional tissue body, the method comprising:
a step of obtaining a cell-containing gel composition by bringing at least one selected from the group consisting of an extracellular matrix component and a fragmented extracellular matrix component, cells, and an ion of a metal element into contact with each other in an aqueous medium; and
a step of culturing the cell-containing gel composition.

10. The production method for a three-dimensional tissue body according to claim 9, wherein the step of obtaining the cell-containing gel composition includes bringing a suspension containing the at least one selected from the group consisting of an extracellular matrix component and a fragmented extracellular matrix component, the cells, and a first aqueous medium into contact with a solution containing the ion of the metal element and a second aqueous medium.

11. The production method for a three-dimensional tissue body according to claim 9 or 10, wherein the step of culturing is performed under a condition in which at least some of the cells maintain a viable state.

12. The production method for a three-dimensional tissue body according to any one of claims 9 to 11, the method further comprising a step of incubating the cell-containing gel composition at 20°C to 30°C after the step of obtaining the cell-containing gel composition and before the step of culturing.

13. The production method for a three-dimensional tissue body according to any one of claims 9 to 12, wherein the metal element is at least one metal element selected from the group consisting of transition metal elements and base metal elements.

14. A three-dimensional tissue body composition comprising:
at least one selected from the group consisting of an extracellular matrix component and a fragmented extracellular matrix component;
cells; and
an ion of a metal element,
wherein the three-dimensional tissue body is transparent at 37°C.

15. The three-dimensional tissue body according to claim 14, wherein a total light transmittance at 37°C is 80% or more.

16. The three-dimensional tissue body according to claim 14 or 15, wherein the metal element is at least one metal element selected from the group consisting of transition metal elements and base metal elements.
